# EUROPEAN PATENT APPLICATION

(11) **EP 4 393 948 A1**
(43) Date of publication of application: **03.07.2024**
(21) Application number: 22216931.0
(22) Date of filing: 28.12.2022
(51) Int. Cl.: C07K 16/24, A61P 17/06, A61P 37/02, A61P 29/00

(54) **THERAPEUTIC VHH ANTIBODIES CROSS-NEUTRALIZING THE IL-17A AND IL-17F HOMODIMERS AS WELL AS THE IL-17AF HETERODIMER**

(71) Applicant: Max-Planck-Gesellschaft zur Förderung der Wissenschaften e.V., 80539 München (DE)
(72) Inventor: The designation of the inventor has not yet been filed
(74) Representative: Weickmann & Weickmann PartmbB

(57) **Abstract**

The present invention pertains in the fields of antibody technology, medicine, pharmacology, infection biology, and medical diagnostics. More specifically, the present disclosure provides VHH antibodies that each potently neutralize the pro-inflammatory interleukin IL-17 in its IL-17A and IL-17F homodimeric as well as in its IL-17AF heterodimeric forms.

## Description

### Field of the invention

The present invention pertains in the fields of antibody technology, medicine, pharmacology, infection biology, and medical diagnostics. More specifically, the present disclosure provides VHH antibodies that each potently neutralize the proinflammatory interleukin IL-17 in its IL-17A and IL-17F homodimeric as well as in its IL-17AF heterodimeric forms.

### Background of the invention

Interleukin 17 (IL-17) is a multifunctional cytokine. It is produced by a specific subclass of CD4-positive T helper cells, i.e., Th17 cells, but also by other CD4- or CD8-positive T cells and gamma/delta T cells. A major effect of IL-17 consists of the attraction of neutrophils and monocytes, and the production of additional cytokines and chemokines by its target cells. Taken together, IL-17 is a key mediator of inflammatory responses. These responses can be of strong benefit to a patient, e.g., when building a defense against various types of infections (Mills, 2022). However, excessive or uncontrolled production of IL-17 can also provoke or exacerbate autoimmune diseases such as psoriasis, rheumatoid arthritis, asthma, or inflammatory bowel diseases.

Upstream signaling pathways tightly control the synthesis and secretion of IL-17 upon mucosal infections. Most notably, Interleukin 23 (IL-23) and IL-1 beta, released from activated dendritic cells or macrophages, stimulate Th17 cells to release IL-17.

Target cells of IL-17 can vary since the receptors are found in a wide array of human cell types. Accordingly, the response can be heterogeneous, too. Most importantly, however, IL-17-exposed epithelial cells release chemokines such as CXCL1 and CXCL8, thereby attracting neutrophilic granulocytes and macrophages to maintain the homeostasis of the epithelial barrier.

In autoimmune diseases, elevated IL-17 levels at the site of inflammation activate the release of Interleukin-6, Tumor Necrosis Factor Alpha (TNF-alpha), and matrix metalloproteinases (MMPs). This leads to tissue damage and amplifies the inflammatory response (Mills, 2022).

IL-17 proteins form dimers. They comprise a family of currently six members, IL-17A through IL-17F. Of these, IL-17A and IL-17F are considered the major and most relevant ones; they are also the best-studied ones. They bind to five receptors, IL-17RA-E, forming heterodimeric receptor complexes. IL-17A and IL-17F bind and activate the IL-17RA and IL-17RC receptors as homodimers or as a heterodimer IL-17A/F (Nies and Panzer, 2020). IL-17 binding requires the adaptor protein Act1 and can lead to the lateral assembly of the receptors, at least partially explaining the onset of intracellular signaling, such as the activation of NF-kappa-B and Mitogen-Activated Protein (MAP) kinases (Wilson *et al.,* 2022).

A number of approaches were developed to interfere with the production and/or activities of IL-17. In particular, monoclonal antibodies can target the upstream IL-23 but, most importantly, IL-17 itself or its receptor. The neutralization of IL-17 then dampens the inflammatory response, which is of benefit when treating autoimmune diseases.

Antagonists to IL-17, particularly antibodies, were successfully applied to treat a number of diseases, giving rise to FDA-approved drugs. Most notably, inflammatory skin diseases, i.e., psoriasis and psoriatic arthritis, as well as Hidradenitis suppurativa ("inverse acne"), were treated successfully by IL-17 neutralizing antibodies (summarized in Skroza *et al.,* 2017; Mills, 2022). Therapeutics include Ixekizumab and Secukinumab that target IL-17A alone in plaque psoriasis (Langley *et al.,* 2014) and psoriatic arthritis (Mease *et al.,* 2015) as well as Bimekizumab against both IL-17A and IL-17F in plaque psoriasis (Reich *et al.,* 2021; Warren *et al.,* 2021) and psoriatic arthritis (Glatt *et al.,* 2018).

However, such antibodies are typically applied systemically, with little or no spatial restriction to the actual sites of inflammation. It should be noted in this context that such indiscriminate block of IL-17 can also exacerbate bacterial or fungal infections as, e.g., reported for Bimekizumab (Reich *et al.,* 2021; Warren *et al.,* 2021). Ideally, the neutralization of IL-17, when treating autoimmune diseases, should be locally confined to the site of the disease rather than applied to the whole body. With a more compact, thermostable, and versatile antibody version, topical applications may become feasible.

Nanobodies (VHH antibodies) comprise a novel class of therapeutic proteins based on monovalent, camelid-derived heavy-chain-only antibodies. In contrast to monoclonal IgGs, which are manufactured in mammalian cells, nanobodies can be produced in bacteria or yeast. Apart from their low molecular weight, certain nanobodies proved to be hyper-thermostable and displayed a particularly high affinity (Güttler *et al.,* 2021).
One IL-17 antagonist, Sonelokimab, was engineered based on single-chain antibodies/nanobodies. It is a trimer of nanobodies targeting IL-17A, IL-17F, and albumin. The idea is to absorb both versions of IL-17 and increase the half-life of the antagonist by binding to albumin. However, this compound, based on its size and albumin-binding entity, is still suitable for systemic rather than topical application. Similar to the classical antibody treatments, the study participants had an increased incidence of fungal Candida infections (Papp *et al.,* 2021).

WO 2012/156219 discloses single domain antibodies that specifically bind to human IL-17A, human IL-17F and/or human IL-17A/F, though the cross-reaction of individual VHHs with these IL-17 isoforms was rather weak.

On top of skin diseases, other inflammatory disorders might become amenable to treatment with IL-17 antagonists. These include but are not limited to ankylosing spondylitis (with approved application), but also Multiple Sclerosis, Rheumatoid arthritis, Asthma, Graft-versus-Host Disease, and even diseases like Alzheimer's', Fatty Liver Disease, and COVID-19 (all clinical studies ongoing as reviewed in Mills, 2022). Future applications may consist of the treatment of autism, Parkinson's Disease, atherosclerosis, stroke, and sepsis. Most of these applications will benefit from a more versatile type of antibody with high affinity, stability, and versatility regarding application routes and fusion to additional/stabilizing entities. This illustrates the immense spectrum of benefits expected from VHH antibodies targeting IL-17A/F.

### Summary of the invention

The present disclosure provides single-domain VHH antibodies that neutralize human IL-17 for treating patients with inflammatory and/or immune-related disorders caused by and/or associated with an overactivity of this cytokine. In certain embodiments, these VHH antibodies are in monovalent form, e.g., as a single VHH domain or as a fusion to a heterologous protein such as serum albumin useable in a wide range of formats. In certain embodiments, The VHH antibodies are in multivalent form, e.g., as bivalent Fc-fusion. The use in a monovalent format is possible due to their very high affinity. In certain embodiments, the VHH antibodies have - in the monovalent format - a target affinity in picomolar or even low picomolar. In certain embodiments, the VHH antibodies neutralizes all major IL-17 isoforms, particularly human IL-17A homodimers, human IL-17F-homodimers and human IL-17A/F heterodimers with similar high potency.

The sequences of the variable regions (CDRs) of preferred VHH antibodies of the present invention as well as assignment of SEQ ID NOs for VHHs and CDRs are listed in the following Table 1

| **VHH** | **Seq ID No.** | **CDR1** | **Seq ID No.** | **CDR2** | **Seq ID No.** | **CDR3** | **Seq ID No.** |
|---|---|---|---|---|---|---|---|
| Re42H11 | **5** | AASGLTASSYAMGWF | **6** | FVAAISWISGGTKYADSVK | **7** | NDMPYGLDTRMDEYAYWG | **8** |
| Bm18A09 | **9** | AASGLTGSSYAMGWF | **10** | FVAAISWISGSTKYTDSVK | **11** | NDMPYGLDTRMDEYVYWG | **12** |
| Re42B03 | **13** | AASGLTGSSYAMGWF | **14** | FVAAISWISGGTKYTDSVK | **15** | NDMPYGLDTRMDEYVYWG | **16** |
| Re42B04 | **17** | EVSGLPVSSYAMGWF | **18** | FVAAISWIGGDSRYAHFVK | **19** | NDMPYGLDTRMDEYEYWG | **20** |
| Bm18F11 | **21** | AASGLTGSSYAMGWF | **22** | FVAAISWISGDTKYTDSVK | **23** | NDMPYGLDTRMDEYVYWG | **24** |
| Bm18B05 | **25** | AVSGRTFSSYAMGWF | **26** | FVAAISWSGGDTKYADFVT | **27** | NDMPYGLDTRMDEYEYWG | **28** |
| Bm17B02 | **29** | VVSGHTSEISAMGWY | **30** | LVALITSRSGDTHYSDSVK | **31** | VHNEPGHLYMA | **32** |
| Re42F08 | **33** | VVSGHTSEISAMGWY | **34** | LVALITSRSGDTHYSDSVK | **35** | VHNEPGHLYMA | **36** |

A list of complete sequences of VHH antibodies is shown at the end of the specification.

A further aspect of the present invention relates to a set of two or more different VHH antibodies, wherein at least one VHH antibody is as described above.

A VHH antibody described above is suitable for use in medicine, e.g., human medicine, particularly for use in therapy, e.g., in the prevention or treatment of a disorder caused by and/or associated with an overactivity of IL-17, particularly an overactivity of IL-17A and/or IL-17F, or in diagnostics, e.g., for detecting IL-17 in a patient sample, e.g., in a body fluid or tissue sample, or in research.

Still a further aspect of the invention relates to a nucleic acid molecule encoding a VHH antibody as described above, particularly in operative linkage with a heterologous expression control sequence, a vector comprising said nucleic acid molecule or a recombinant cell or non-human organism transformed or transfected with said nucleic acid molecule or said vector.

Still a further aspect of the invention relates to a method for recombinant production of a VHH antibody as described above, comprising cultivating a cell or an organism as described above in a suitable medium and obtaining the VHH antibody from the cell or organism or from the medium.

Still a further aspect of the invention relates to a method for the prevention or treatment of a disorder caused by and/or associated with an overactivity of IL-17, particularly an overactivity of IL-17A and/or IL-17F, comprising administering an effective dose of the VHH antibody as described above or the set of at least two different VHH antibodies as described above to a subject in need thereof, particularly to a human subject from suffering from a disorder caused by and/or associated with IL-17.

A non-limitative list of VHH antibodies of the present invention and their utility for IL-17 neutralization is shown in the following Table 2:

**Table 2: Properties of disclosed anti-IL-17 VHH antibodies.**

| VHH | Class | Seq. ID | KDs (pM) | | | Thermostability | IP | IL-17 Neutralization |
|---|---|---|---|---|---|---|---|---|
| | | | IL-17A | IL-17A/F | IL-17F | | | |
| Re42H11 | Re42H11 | 5 | ≤ 10 | ≤ 10 | ≤ 10 | ≥95°C | 5.8 | Yes |
| Bm18A09 | Re42H11 | 9 | | | | | | |
| Re42B03 | Re42H11 | 13 | ≤ 10 | ≤ 10 | ≤ 10 | ≥95°C | 5.0 | Yes |
| Re42B04 | Re42H11 | 17 | ≤ 10 | ≤ 10 | ≤ 10 | 57°C (partial melting) | 4.8 | Yes |
| Bm18F11 | Re42H11 | 21 | ≤ 10 | ≤ 10 | ≤ 10 | ≥95°C | 4.7 | Yes |
| Bm18B05 | Re42H11 | 25 | ≤ 10 | ≤ 10 | ≤ 10 | ≥95°C | 4.5 | Yes |
| Bm17B02 | Bm17B02 | 29 | ≤ 10 | ≤ 10 | 70 | ≥95°C | 6.9 | Yes |
| Re42F08 | Bm17B02 | 33 | ≤ 10 | ≤ 10 | ≤ 10 | ≥95°C | 7.2 | Yes |

### Embodiments of the invention

In the following, specific embodiments of the invention are disclosed as follows:
1. A VHH antibody recognizing a human IL-17 polypeptide,
   which cross-reacts with a plurality of different human IL-17 polypeptides comprising (i) a human IL-17A homodimer, (ii) a human IL-17F homodimer and (iii) a human IL17A/F heterodimer wherein the binding affinity expressed as dissociation constant KD to each of (i), (ii), and (iii) is about 1 nM or less, about 100 pM or less, about 50 pM or less, about 20 pM or less or about 10 pM or less.
2. The VHH antibody according to embodiment 1,
   which competes with the VHH antibody Re42H11 having a VH sequence as shown in SEQ. ID NO: 5 for the binding to a human IL-17 polypeptide.
3. The VHH antibody according to embodiment 1,
   which competes with the VHH antibody Bm17B02 having a VH sequence as shown in SEQ. ID NO: 29 for the binding to a human IL-17 polypeptide.
4. A VHH antibody recognizing a human IL-17 polypeptide, comprising
   (a) a CDR3 sequence as shown in SEQ. ID NO: 6, 10, 14, 18, 22, 26, 30 and 34;
   (b) a CDR3 sequence, which has an identity of at least 80%, at least 90% or at least 95% to a CDR3 sequence of (a), or
   (c) a VHH antibody, which competes with the VHH antibody Re42H11 having a VHH sequence as shown in SEQ. ID NO: 5 for the binding to a human IL-17 polypeptide, or which competes with the VHH antibody Bm17B02 having a VHH sequence as shown in SEQ. ID NO: 29 for the binding to a human IL-17 polypeptide.
5. The VHH antibody according to embodiment 4 comprising
   (a) a combination of CDR1, CDR2 and CDR3 sequences as shown in SEQ. ID NO: 6-8, 10-12, 14-16, 18-20, 22-24, 26-28, 30-32, and 34-36,
   (b) a combination of CDR1, CDR2 and CDR3 sequences which has an identity of at least 80%, at least 90% or at least 95% to a combination of CDR1, CDR2 and CDR3 sequences of (a), or
   (c) a VHH antibody, which competes with the VHH antibody Re42H11 having a VH sequence as shown in SEQ. ID NO: 5 for the binding to a human IL-17 polypeptide, or which competes with the VHH antibody Bm17B02 having a VH sequence as shown in SEQ. ID NO: 29 for the binding to a human IL-17 polypeptide.
6. The VHH antibody according to any one of the previous embodiments, comprising
   (a) a VHH sequence as shown in SEQ. ID NO: 5, 9, 13, 17, 21, 25, 29, or 33;
   (b) a sequence, which has an identity of at least 80%, at least 90%, at least 95% or at least 99% to a VHH sequence of (a), or
   (c) a VHH antibody, which competes with the VHH antibody Re42H11 having a VHH sequence as shown in SEQ. ID NO: 5 for the binding to a human IL-17 polypeptide, or which competes with the VHH antibody Bm17B02 having a VHH sequence as shown in SEQ. ID NO: 29 for the binding to a human IL-17 polypeptide.
7. The VHH antibody of any one of embodiments 4-6, which cross-reacts with (i) an IL-17A homodimer, (ii) an IL-17F homodimer and (iii) an IL17A/F heterodimer.
8. The VHH antibody of embodiment 7, wherein the binding affinity expressed as dissociation constant KD to each of (i), (ii), and (iii) is about 1 nM or less, about 100 pM or less, about 50 pM or less, about 20 pM or less or about 10 pM or less.
9. The VHH antibody of any one of the preceding embodiments, which neutralizes the binding of a human IL-17 polypeptide to a human IL-17 receptor.
10. The VHH antibody of embodiment 9, which neutralizes the binding of (i) a human IL-17A homodimer, (ii) a human IL-17F homodimer and (iii) a human IL17A/F heterodimer to a human IL-17 receptor.
11. The VHH antibody of any one of embodiments 9-10, which neutralizes the binding of a human IL-17 polypeptide, particularly (i) a human IL-17A homodimer, (ii) a human IL-17F homodimer and (iii) a human IL17A/F heterodimer to a human IL-17 receptor at a concentration of about 10 nM or less, of about 1 nM or less, or of about 0.5 nM or less.
12. The VHH antibody of any one of the preceding embodiments, which is stable, particularly thermostable or hyperthermostable.
13. The VHH antibody of embodiment 12, which has a melting temperature of at least about 65°C, of at least about 80°C, of at least 90°C or of at least about 95°C when measured under non-reducing conditions.
14. The VHH antibody of embodiment 12 or 13, which has an aggregation temperature of at least about 50°C, of at least about 60°C, of at least 70°C or of at least about 80°C when measured under non-reducing conditions.
15. The VHH antibody of any one of embodiments 1-14, which is selected from antibody Re42H11 comprising a VHH sequence as shown in SEQ. ID NO: 5 or a VHH antibody, which is a variant thereof.
16. The VHH antibody of any one of embodiments 1-14, which is selected from antibody Bm18A09 comprising a VHH sequence as shown in SEQ. ID NO: 9 or a VHH antibody, which is a variant thereof.
17. The VHH antibody of any one of embodiments 1-14, which is selected from antibody Re42B03 comprising a VHH sequence as shown in SEQ. ID NO: 13 or a VHH antibody, which is a variant thereof.
18. The VHH antibody of any one of embodiments 1-14, which is selected from antibody Re42B04 comprising a VHH sequence as shown in SEQ. ID NO: 17 or a VHH antibody, which is a variant thereof.
19. The VHH antibody of any one of embodiments 1-14, which is selected from antibody Bm18F11 comprising a VHH sequence as shown in SEQ. ID NO: 21 or a VHH antibody, which is a variant thereof.
20. The VHH antibody of any one of embodiments 1-14, which is selected from antibody Bm18B05 comprising a VHH sequence as shown in SEQ. ID NO: 25 or a VHH antibody, which is a variant thereof.
21. The VHH antibody of any one of embodiments 1-14, which is selected from antibody Bm17B02 comprising a VHH sequence as shown in SEQ. ID NO: 29 or a VHH antibody, which is a variant thereof.
22. The VHH antibody of any one of embodiments 1-14, which is selected from antibody Re42F08 comprising a VHH sequence as shown in SEQ. ID NO: 33 or a VHH antibody, which is a variant thereof.
23. The VHH antibody of any one of the previous embodiments, which is non-glycosylated, or which is glycosylated.
24. The VHH antibody of any one of the previous embodiments, which is produced in a bacterium, e.g., E. coli, or in a yeast, e.g., Pichia pastoris, or in a human or animal cell, e.g., a mammalian cell.
25. The VHH antibody of any one of embodiments 1-24, which is in a monovalent format.
26. The VHH antibody of any one of embodiments 1-24, which is in a multimeric format.
27. The VHH antibody of embodiment 26, which is in a dimeric format.
28. The VHH antibody of any one of embodiments 1-27, which is covalently or non-covalently conjugated to a heterologous moiety, e.g., a labeling group, a capture group or an effector group, wherein the heterologous moiety is particularly selected from a fluorescence group, biotin, an enzyme such as a peroxidase, phosphatase or luciferase, a hapten, an affinity tag, or a nucleic acid such as an oligonucleotide.
29. The VHH antibody of any one of embodiments 1-28, which is fused to a heterologous polypeptide moiety, or which is fused to an Fc Fragment, or which is fused to serum albumin or an albumin-binding moiety.
30. The VHH antibody of any of embodiments 1-28, which is conjugated to one or several non-peptidic polymer moieties, preferably hydrophilic polymer moieties, such as polyethylene glycol (PEG).
31. A set of two or more different VHH antibodies recognizing a human IL-17 polypeptide, particularly an IL-17A homodimer, an IL-17F homodimer and an IL17A/F heterodimer, comprising at least one VHH antibody of any of embodiments 1-30, particularly a VHH antibody in a monovalent format.
32. The VHH antibody of any one of embodiments 1-30 or the set of embodiment 31 for use in medicine, particularly for use in therapy or diagnostics.
33. The VHH antibody of any one of embodiments 1-30 or the set of embodiment 31 for use in the prevention or treatment of a disorder caused by and/or associated with an overactivity of IL-17, particularly with an overactivity of IL-17A and/or IL-17F.
34. The VHH antibody of any one of embodiments 1-30 or the set of embodiment 31 for use in the prevention or treatment of an inflammatory and/or immune-related disorder.
35. The VHH antibody of any one of embodiments 1-30 or the set of embodiment 31 for use in the prevention or treatment of an inflammatory and/or immune-related skin disorder.
36. The VHH antibody of any one of embodiments 1-30 or the set of embodiment 31 for use in the prevention or treatment of asthma, psoriasis, e.g., plaque psoriasis, arthritis, e.g., rheumatoid arthritis or psoriatic arthritis, Hidradenitis suppurativa, inflammatory bowel disease (Crohn's disease, ulcerative colitis), multiple sclerosis, skin cancer, ankylosing spondylitis, uveitis, atopic dermatitis, Graft versus Host Disease, Alzheimer's disease, fatty liver disease, COVID-19, sepsis, ischemic stroke, Parkinson's disease, influenza virus infection.
37. The VHH antibody of any one of embodiments 1-30 or the set of embodiment 31 for use according to any one of embodiments 32-36 in a human subject.
38. The VHH antibody of any one of embodiments 1-30 or the set of embodiment 31 for use according to any one of embodiments 32-36, which is administered locally.
39. The VHH antibody of any one of embodiments 1-30 or the set of embodiment 31 for use according to any one of embodiments 32-36, which is administered topically.
40. A pharmaceutical composition comprising as an active agent a VHH antibody of any one of embodiments 1-30 or the set of embodiment 31, and a pharmaceutically acceptable carrier.
41. A nucleic acid molecule encoding a VHH antibody or a subunit of a VHH antibody according to any one of embodiments 1-30, preferably in operative linkage with a heterologous expression control sequence.
42. A vector comprising a nucleic acid molecule according to embodiment 41.
43. A recombinant cell or non-human organism transformed or transfected with a nucleic acid molecule according to embodiment 40 or a vector according to embodiment 42.
44. The cell or organism of embodiment 43, which is selected from a bacterium such as E. *coli Bacillus sp.,* a unicellular eukaryotic organism, e.g., yeast such as *Pichia pastoris,* or *Leishmania,* an insect cell, a mammalian cell, or a plant cell.
45.A method for recombinant production of a VHH antibody of any one of embodiments 1-30, comprising cultivating a cell or an organism of embodiment 43 or 44 in a suitable medium and obtaining the VHH antibody from the cell or organism or from the medium.
46. The method of embodiment 45, comprising cultivating a yeast such as *Pichia pastoris* and obtaining the VHH antibody from the medium.
47. A method for the prevention or treatment of a disorder caused by and/or associated with an overactivity of IL-17comprising administering an effective dose of the VHH antibody of any one of embodiments 1-30, the set of embodiment 31, or the pharmaceutical composition of embodiment 40 to a subject in need thereof, particularly to a human subject.

### Detailed Description

The present invention relates to a VHH antibody recognizing a human IL-17 polypeptide.

### VHH antibodies

The present invention relates to a VHH antibody, which is a monovalent heavy chain-only antibody comprising a CDR1 domain, a CDR2 domain and a CDR3 domain linked by framework regions including, but not being limited to, whole VHH antibodies, e.g. native VHH antibodies comprising framework regions derived from camelids, and modified VHH antibodies comprising modified framework regions, VHH antibody fragments and VHH antibody fusion proteins, e.g. a fusion protein with an immunoglobulin or non-immunoglobulin peptide or polypeptide, as long as it shows the properties according to the invention.

There are several methods known in the art for determining the CDR sequences of a given antibody molecule, but there is no standard unequivocal method. Determination of CDR sequences from antibody heavy chain variable regions can be made according to any method known in the art, including, but not limited to, the methods known as Kabat, Chothia, and IMGT. A selected set of CDRs may include sequences identified by more than one method, namely, some CDR sequences may be determined using Kabat and some using IMGT, for example. According to some embodiments of the present invention, the CDR sequences of a VHH variable region are determined using the Kabat method. CDRs may also be defined through a multiple alignment (with many other VHH antibodies), to identify the hot-spots of variability and relate them to a standard VHH antibody structure. It is also possible to define CDRs by analyzing the structure of the VHH antibody and deciding what is a loop and what is the antibody's scaffold. In some cases, CDR-adjacent residues are also variable, and are therefore included in the CDR definition.

The present invention is also directed to a covalent or non-covalent conjugate of a VHH antibody molecule to a non-proteinaceous structure, for example, a labeling group, a capture group such a solid phase-binding group, or an effector group such as a toxin. For example, the heterologous moiety may be from a fluorescence group, biotin, an enzyme such as a peroxidase, phosphatase, or luciferase, a hapten, an affinity tag, or a nucleic acid such as an oligonucleotide.

The VHH antibody of the present invention is particularly a monoclonal VHH antibody characterized by a specific amino acid sequence. The VHH antibody may be produced in a prokaryotic host cell, a yeast cell or a mammalian cell. In certain embodiments, the VHH antibody is non-glycosylated. In certain embodiments, the VHH antibody is glycosylated, wherein a carbohydrate structure may be derived from a glycosylation site introduced into the VHH sequence and/or from a fusion partner.

A VHH antibody according to the present invention is characterized by (i) a CDR3 sequence, (ii) a combination of a CDR1 sequence, a CDR2 sequence, and a CDR3 sequence, (iii) by a complete VHH sequence, or (iv) by competition with a specific reference antibody. Specific CDR and VHH sequences are provided in the Tables, Figures and the Sequence Listing.

According to the present invention, sequences related to the above sequences are encompassed. These related sequences are defined by having a minimum identity to a specifically indicated amino acid sequence, e.g., a CDR or VHH sequence. This identity is indicated over the whole length of the respective reference sequence and may be determined by using well-known algorithms such as BLAST.

In particular embodiments, a related CDR3 sequence has an identity of at least 80% or at least 90% or at least 95% to a specifically indicated CDR3 sequence, e.g., a substitution of 1, 2, or 3 amino acids.

In particular embodiments, a related combination of a CDR1 sequence, a CDR2 sequence, and a CDR3 sequence has an identity of at least 80% or at least 90% or at least 95% to a specifically indicated combination of a CDR1 sequence, a CDR2 sequence, and a CDR3 sequence, e.g., a substitution of 1, 2, 3, 4, 5 or 6 amino acids by different amino acids.

In particular embodiments, a related VHH sequence has an identity of least 70%, at least 80%, at least 90%, at least 95% or at least 99% to a VHH sequence, e.g., a substitution of 1, 2, 3, 4, 5 or up to 20 amino acids.

Further, the invention refers to a VHH antibody, which competes with a specific VHH antibody disclosed herein for the binding to a human IL-17 polypeptide. In certain embodiments, a competing VHH antibody binds the same or an overlapping epitope on the human IL-17 polypeptide. For example, the invention refers to a VHH antibody, which competes either with a reference antibody, e.g., VHH antibody Re42H11 or with VHH antibody Bm17B02. Competition may be determined by label-free biolayer interferometry performed as a cross-competition or epitope binning assay using a label-free detection system, e.g., an Octet^{®} system from Sartorius, according to the manufacturer's instructions.

In particular embodiments, at least one amino acid of a reference sequence, including an amino acid in a CDR1, CDR2 or CDR3 sequence and/or an amino acid in a framework region, is replaced by another amino acid, while preserving structural integrity and epitope-binding of the VHH antibody. These exchanges can be conservative (i.e., by a similar amino acid) or non-conservative.

In further particular embodiments, at least one amino acid of a reference sequence, including an amino acid in a CDR1, CDR2 or CDR3 sequence and/or an amino acid in a framework region, is replaced by a conservative amino acid substitution, i.e. a substitution of an amino acid by another amino acid with similar biochemical properties, for example a substitution of an aliphatic amino acid, e.g. Gly, Ala, Val, Leu, or Ile, for another aliphatic amino acid; a substitution of a basic amino acid, e.g. His, Lys or Arg, against another basic amino acid or against Met; a substitution of an acidic amino acid or an amide thereof, e.g., Asp, Glu, Asn or Gln, against another acidic amino acid or an amide thereof; a substitution of an aromatic amino acid, e.g., Phe, Tyr or Trp, against another aromatic amino acid.

In further particular embodiments, the VHH antibody is selected from antibody Re42H11 comprising a VHH sequence as shown in SEQ. ID NO: 5 or a VHH antibody, which is a variant thereof. In certain embodiments, 1, 2, 3, 4, 5, 6, 7, 8, 9 or 10 amino acids of SEQ. ID NO: 5 are replaced by another amino acid.

In further particular embodiments, the VHH antibody is selected from antibody Bm18A09 comprising a VHH sequence as shown in SEQ. ID NO: 9 or a VHH antibody, which is a variant thereof. In certain embodiments, 1, 2, 3, 4, 5, 6, 7, 8, 9 or 10 amino acids of SEQ. ID NO: 9 are replaced by another amino acid.

In further particular embodiments, the VHH antibody is selected from antibody Re42B03 comprising a VHH sequence as shown in SEQ. ID NO: 13 or a VHH antibody, which is a variant thereof. In certain embodiments, 1, 2, 3, 4, 5, 6, 7, 8, 9 or 10 amino acids of SEQ. ID NO: 9 are replaced by another amino acid.

In further particular embodiments, the VHH antibody is selected from antibody Re42B04 comprising a VHH sequence as shown in SEQ. ID NO: 17 or a VHH antibody, which is a variant thereof. In certain embodiments, 1, 2, 3, 4, 5, 6, 7, 8, 9 or 10 amino acids of SEQ. ID NO: 13 are replaced by another amino acid.

In further particular embodiments, the VHH antibody is selected from antibody Bm18F11 comprising a VHH sequence as shown in SEQ. ID NO: 21 or a VHH antibody, which is a variant thereof. In certain embodiments, 1, 2, 3, 4, 5, 6, 7, 8, 9 or 10 amino acids of SEQ. ID NO: 17 are replaced by another amino acid.

In further particular embodiments, the VHH antibody is selected from antibody Bm18B05 comprising a VHH sequence as shown in SEQ. ID NO: 25 or a VHH antibody, which is a variant thereof. In certain embodiments, 1, 2, 3, 4, 5, 6, 7, 8, 9 or 10 amino acids of SEQ. ID NO: 21 are replaced by another amino acid.

In further particular embodiments, the VHH antibody is selected from antibody Bm17B02 comprising a VHH sequence as shown in SEQ. ID NO: 29 or a VHH antibody, which is a variant thereof. In certain embodiments, 1, 2, 3, 4, 5, 6, 7, 8, 9 or 10 amino acids of SEQ. ID NO: 25 are replaced by another amino acid.

In further particular embodiments, the VHH antibody is selected from antibody Re42F08 comprising a VHH sequence as shown in SEQ. ID NO: 33 or a VHH antibody, which is a variant thereof. In certain embodiments, 1, 2, 3, 4, 5, 6, 7, 8, 9 or 10 amino acids of SEQ. ID NO: 29 are replaced by another amino acid.

Further, the present invention relates to a nucleic acid molecule, e.g., a DNA molecule, encoding a VHH as indicated above, or a vector, comprising said nucleic acid molecule as indicated above in operative linkage with an expression control sequence, particularly with a heterologous expression control sequence. Furthermore, the invention relates to a cell comprising a nucleic acid molecule or a vector as described above. Vectors for the recombinant production of VHH antibodies are well-known in the art. In certain embodiments, the vector is an extrachromosomal vector. In other embodiments, the vector is a vector for genomic integration. The cell may be a known host cell for producing antibodies or antibody fragments, e.g., a prokaryotic cell such as an E. coli or a Bacillus sp. cell, a yeast cell, particularly a *Pichia* yeast cell, an insect cell or a mammalian cell, e.g., a CHO cell, or a plant cell. In certain embodiments, the cell comprises the nucleic acid or the vector extrachromosomally. In other embodiments, the cell comprises the nucleic acid or the vector integrated into the genome, e.g., as a genomically integrated expression cassette.

Still a further aspect of the present invention is a method of recombinantly producing a VHH antibody by growing a cell as described above in a culture medium and obtaining the VHH antibody from the cell or the culture medium. Suitable culture media and culture conditions are well known in the art.

### Binding to human IL17

The VHH antibodies of the present invention bind to a human IL.-17 polypeptide. The inventors have identified VHH antibodies, which bind with high affinity to human IL-17 polypeptides and cross-react with (i) a human IL-17A homodimer, (ii) a human IL-17F homodimer and (iii) a human IL17A/F heterodimer as shown in Table 1, supra.

In the context of the present disclosure the term "human IL-17". This term encompasses a plurality of different human IL-17 family members including but not limited to:
- IL-17A (UniProt Accession No. Q16552)
- IL-17B (UniProt Accession No. Q9UHF)
- IL-17C (UniProt Accession No. Q9P0M4)
- IL-17D (UniProt Accession No. Q8TAD2)
- IL-17E (UniProt Accession No. Q9H293)
- IL-17F (UniProt Accession No. Q96PD4)

In preferred embodiments, the term "human IL-17" encompasses human IL-17A (UniProt Accession No. Q16552) particularly in the form of a homodimer comprising two IL-17A units, IL-17F (UniProt Accession No. Q96PD4) particularly in the form of a homodimer comprising 2 IL-17F units, and IL-17AF in the form of a heterodimer comprising one IL-17A unit and one IL-17F unit.

It should be noted, however, that the term "human IL17" also encompasses naturally occurring variants of human IL-17 polypeptides and genetically modified constructs as described herein.

The inventors have performed their selection and binding experiments with genetically modified IL-17A, IL-17F and IL-17A/F constructs as follows:
- An IL-17A homodimer that comprises amino acid residues 40-155 of human IL-17A (UniProt Q16552). This corresponds to the portion that is well-ordered in crystal structures. The first five amino acids (GSEDS) are spacer residues. Further, it comprises two point mutations: N68D (eliminates an N-glycosylation site) and C192S (eliminates an unpaired cysteine that would otherwise cause problems in recombinant expression). The amino acid sequence of this polypeptide is shown in SEQ. ID NO: 1.
- An IL-17F homodimer that comprises amino acid residues 39-163 of human IL-17F (UniProt Q96PD4). N83D mutation to eliminate an N-glycosylation site. The first five residues (GSEGE) are a linker. The amino acid sequence of this polypeptide is shown in SEQ. ID NO: 2.
- An IL17A/F heterodimer that consists of two polypeptides. The IL-17A unit comprises amino acid residues 40-155 of human IL-17A (UniProt Q16552) with 5 spacer residues (GSEDS) in front and a N68D mutation. The amino acid sequence of this polypeptide is shown in SEQ. ID NO: 3. The IL-17F unit comprises amino acid residues 39-163 of human IL-17F (UniProt Q96PD4) with 5 spacer residues (GSEDS) in front and N83D and C137S mutations. The amino acid sequence of this polypeptide is shown in SEQ. ID NO: 4.

In certain embodiments, the VHH antibody of the present invention binds to (i) a human IL-17A homodimer, (ii) a human IL-17F homodimer and (iii) a human IL17A/F heterodimer wherein the binding affinity expressed as dissociation constant KD to each of (i), (ii), and (iii) is about 1 nM or less, about 100 pM or less, about 50 pM or less, about 20 pM or less or about 10 pM or less. The binding affinity may be determined as described herein in detail in the Examples using the polypeptides of SEQ: ID NO: 1-4 as described above.

### IL-17 neutralization

The VHH antibodies of the present invention are capable of neutralizing the binding of human IL-17 to a human IL-17 receptor. The inventors have identified VHH antibodies, which cross-neutralize (i) a human IL-17A homodimer, (ii) a human IL-17F homodimer and (iii) a human IL17A/F heterodimer as shown in Table 1, supra.

In certain embodiments, the VHH antibody of the present invention cross-neutralizes the binding of a human IL-17 polypeptide, particularly (i) a human IL-17A homodimer, (ii) a human IL-17F homodimer and (iii) a human IL17A/F heterodimer to a human IL-17 receptor at a concentration of about 10 nM or less, of about 1 nM or less, or of about 0.5 nM or less. The neutralization potency may be determined as described herein in detail in the Examples.

### Stability

For the intended therapeutic application, the anti-IL-17 VHH antibodies should not only be highly potent in interleukin neutralization, but also, they should be developable as biological drugs. This includes that they are stable enough to survive a lengthy, large-scale production process as well as transportation and storage (ideally for years in liquid formulation) without aggregation or loss of activity.

A good predictor for stability is thermostability, which can be measured, e.g., by thermal shift assays or specifically by differential scanning fluorimetry. The present inventors have identified several thermostable or hyperthermostable VHH antibodies as shown in Table 1, supra.

In a particular embodiment, the invention relates to a VHH antibody, which is stable, particularly thermostable, or hyperthermostable. Preferably, the VHH antibody has a melting point (melting temperature) of at least about 65°C, of at least about 80°C, of at least 90°C or of at least about 95°C, and/or an aggregation temperature of at least about 50°C, of at least about 60°C, of at least 70°C or of at least about 80°C when measured under non-reducing conditions. Melting and aggregation temperatures are determined as described herein.

### Sets of VHH antibodies

In a further aspect, the present invention relates to a set comprising at least 2, 3, 4 or more of the above VHH antibodies. In such a set, the individual VHH antibodies are present in suitable molar ratios. Typically, the molar ratios are in the range of about 2:1 to about 1:2, particularly about 1.5:1 to about 1:1.5, even more particularly about 1:1.

In certain embodiments, the VHH antibody set may comprise a single composition wherein the VHH antibodies in said set consist of a predetermined number of different species of VHH antibodies as described above. The VHH antibody set may comprise a plurality of compositions each comprising a different species of VHH antibody as described above. The set of the present invention may be free from other VHH antibodies.

### Monovalent and multivalent VHH antibodies

In certain embodiments, the VHH antibody of the present invention is in a monovalent format, i.e., it has a single binding site for a human IL-17 polypeptide. In these embodiments, the VHH antibody may be present as such or covalently or non-covalently attached to a heterologous moiety, e.g., a peptidic or non-peptidic moiety.

In further embodiments, the VHH antibody of the present invention is in in a multimeric, e.g., dimeric, or trimeric format. In these embodiments, several VHH antibody units may be covalently or non-covalently attached to each other together via a linker and/or a multimerization, e.g., dimerization or trimerization moiety.

In certain embodiments, the VHH antibody is a homodimeric VHH antibody, wherein a VHH antibody unit is covalently attached to a dimerization moiety, e.g., an immunoglobulin Fc fragment.

In certain embodiments, the VHH antibody is a heterodimeric VHH antibody, particularly a covalently linked VHH heterodimer comprising a first VHH antibody and a second VHH antibody wherein the first VHH antibody and the second VHH antibody bind to different epitopes on IL-17 or wherein the first VHH antibody binds to IL-17 and the second VHH antibody binds to a different target.

### Production of VHH antibodies

VHH antibodies including monomeric and multimeric VHH antibodies may be produced as described in WO 2022/023483 and WO 2022/023484, the contents of which are herein incorporated by reference, or by other methods well known in the art.

A VHH antibody may be recombinantly produced in a suitable host cell, e.g., in a prokaryotic or eukaryotic host cell or host organism. For this purpose, a nucleic acid molecule encoding the VHH antibody is introduced into the host cell or host organism and expressed in the host cell or host organism. The nucleic acid molecule may encode a monomeric VHH antibody or a subunit of a multimeric VHH antibody.

In a particular embodiment, the VHH antibody is recombinantly produced in a bacterium, e.g., E. coli or Bacillus. For example, expression in a bacterium may involve cytoplasmic and/or periplasmic expression and purification of the VHH antibody from the host cell, or secretory expression and purification of the VHH antibody from the culture medium. In certain embodiments, the nucleic acid sequence encoding the VHH antibody is fused to at least one sequence directing the expression to the periplasm and/or into the culture medium.

In a further particular embodiment, the VHH antibody is recombinantly produced in a eukaryotic host cell or host organism, preferably in yeast, e.g., in Pichia pastoris, Saccharomyces cerevisiae, or Hansenula polymorpha, or in an animal cell, particularly in a mammalian, e.g., human or a hamster cell. For example, expression in a eukaryotic host cell or host organism, e.g., yeast may involve cytoplasmic and/or periplasmic expression and purification of the VHH antibody from the host cell, or preferably secretion from the host cell and purification of the VHH antibody from the culture medium. In certain embodiments, the nucleic acid sequence encoding the VHH antibody is fused to at least one sequence directing the expression into the culture medium.

### Therapeutic applications

Still a further aspect of the present invention is the use of a VHH antibody as described above in medicine, particularly for therapeutic and/or in vitro or vivo diagnostic use. In certain embodiments, the VHH antibody is used in human medicine.

The VHH antibody of the present invention is useful in the prevention or treatment of a disorder caused by and/or associated with an overactivity of IL-17, particularly associated with an overactivity of IL-17A and/or IL-17F.

In certain embodiments, the VHH antibody is useful in the prevention or treatment of an inflammatory and/or immune-related disorder, e.g., an inflammatory and/or immune-related skin disorder.

Exemplary disorders are asthma, psoriasis, e.g., plaque psoriasis, arthritis, e.g., rheumatoid arthritis or psoriatic arthritis, Hidradenitis suppurativa, inflammatory bowel disease (Crohn's disease, ulcerative colitis), multiple sclerosis, skin cancer, ankylosing spondylitis, uveitis, atopic dermatitis, Graft versus Host Disease, Alzheimer's disease, fatty liver disease, COVID-19, sepsis, ischemic stroke, Parkinson's disease, influenza virus infection.

In therapeutic applications, the VHH antibody is administered in an effective amount to a subject in need thereof, particularly to a human subject. The dose will depend on the specific type of agent, e.g., monovalent VHH antibody or multimeric VHH antibody, the type of disease, and the route of administration.

Typically, the VHH antibody is administered as a pharmaceutical composition comprising the active agent and a pharmaceutically acceptable carrier or excipient. Examples of suitable carriers and excipients for formulating antibodies or antibody fragments are well-known in the art.

Depending on the stage and the severity of the disorder, the pharmaceutical composition may be administered once or several times during the course of the disorder. For example, it may be administered once or several times daily, each second day, two times weekly or weekly for a suitable period of time.

In certain embodiments, the pharmaceutical composition is administered parenterally, e.g., by subcutaneous, intramuscular or intravenous injection or by infusion. In further embodiments, the pharmaceutical composition is administered locally, e.g., topically, orally, nasally or intrapulmonary, for example by inhalation as an aerosol.

The VHH antibody may be administered alone or together with a further active agent, particularly with a further agent that is useful in the prevention and/or treatment of a disorder caused by and/or associated with IL-17 overactivity, particularly associated with an overactivity of IL-17A and/or IL-17F.

In certain embodiments, the further active agent is an active agent that is useful in the prevention and/or treatment of an exemplary disorder listed above.

### Diagnostic applications

Diagnostic applications include in vitro methods wherein a VHH antibody is used for detecting IL-17 in a sample, e.g., in a body fluid such as saliva, blood, serum or plasma, stool samples or in a tissue or biopsy sample. Diagnostic applications further include in vivo methods wherein a VHH antibody is used for detecting IL-17 in a subject, particularly in a human patient. For diagnostic applications the VHH antibody may carry a label for direct detection or used in combination with secondary detection reagents, e.g., antibodies, including conventional antibodies or VHH antibodies, for indirect detection according to established techniques in the art.

Further, the present invention is explained in more detail by the following Figures and Examples.

### Figure Legends

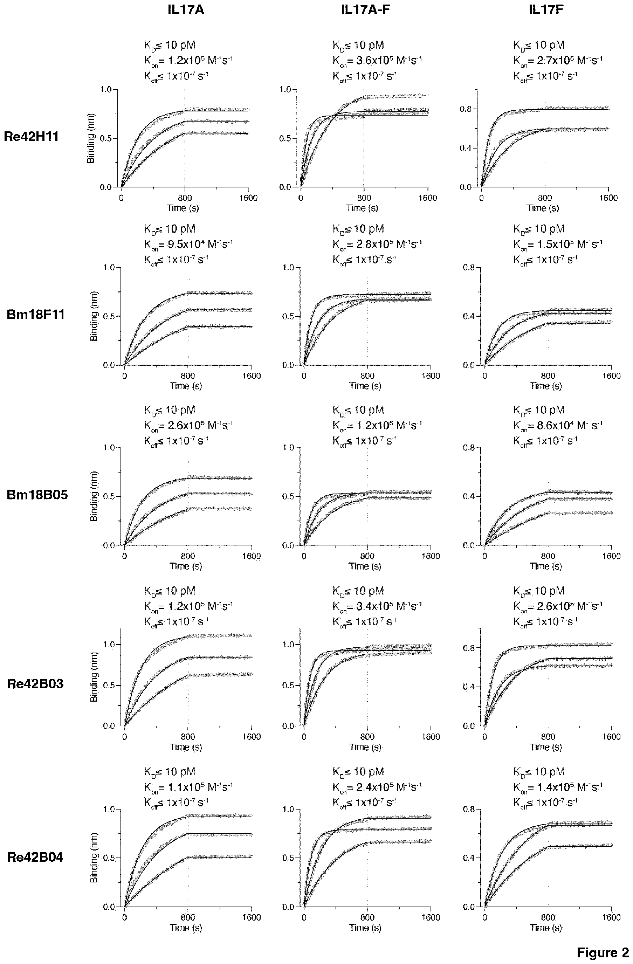

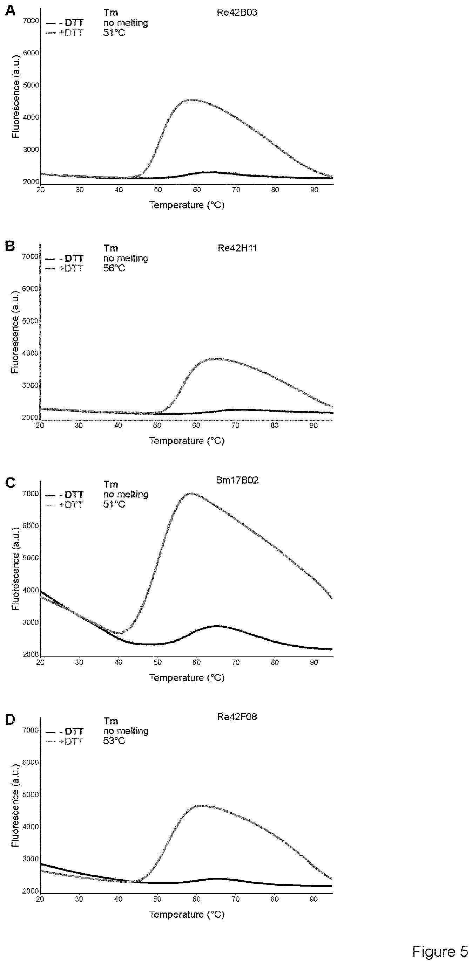

**Figure 1****: Sequence of disclosed anti-IL-17 VHH antibodies.**
   Non-conserved residues are highlighted by a colored background.
**Figure 2****: Affinities of Re42H11 class VHHs for IL-17 isoforms as measured by Bio-layer interferometry (BLI).**
   VHH antibodies were produced with a C-terminal Avi-tag and enzymatically biotinylated by recombinant BirA (Beckett *et al.,* 1999). They were subsequently immobilized at a concentration of 0.7 µg/ml for 110 seconds to High Precision Streptavidin biosensors on an Octet RED96e instrument (ForteBio/Sartorius), using Phosphate-Buffered Saline (PBS) pH 7.4, 0.02% (w/v) Tween 20 and 0.1% (w/v) bovine serum albumin (BSA) as an assay buffer. 10, 20, and 40 nM of indicated IL-17 species were then allowed to bind for 800 seconds and, subsequently, to dissociate for another 800 seconds. Binding and dissociation were recorded as wavelength shifts (in nm). Baselines were recorded by measuring a 'minus VHH control' in parallel. On-rates, off-rates, and dissociation constants (K_{D}s) were calculated by the Octet Data Analysis HT 12.0 software), using a mass transport model to fit the data. Note that all analyzed VHH antibodies bind either IL-17A, IL-17F, or the IL-17AF heterodimer with enhance the fluorescence of the added SYPRO orange dye. Assays were performed in a volume of 20 µl, at 1 mg/ml VHH concentration in 50 mM Tris/HCl, 150 mM NaCl (pH 8.0 at 20°C), and 1x dye (diluted from a 5000x stock; Life Technologies apparently low picomolar K_{D}s.

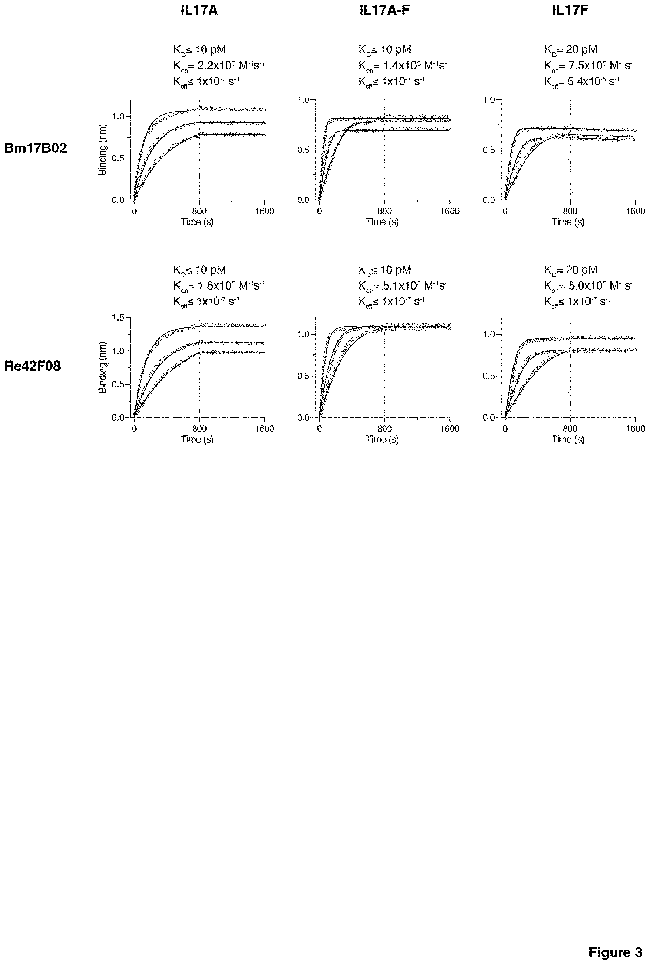

**Figure 3****: Affinities of Bm17B02 class VHHs for IL-17 isoforms.**
   Measurements were performed as described in Figure 2, the difference being that Bm17B02 and Re42F08 were analyzed, which represent the second perfectly IL-17A and IL-17F cross-reacting VHH class.

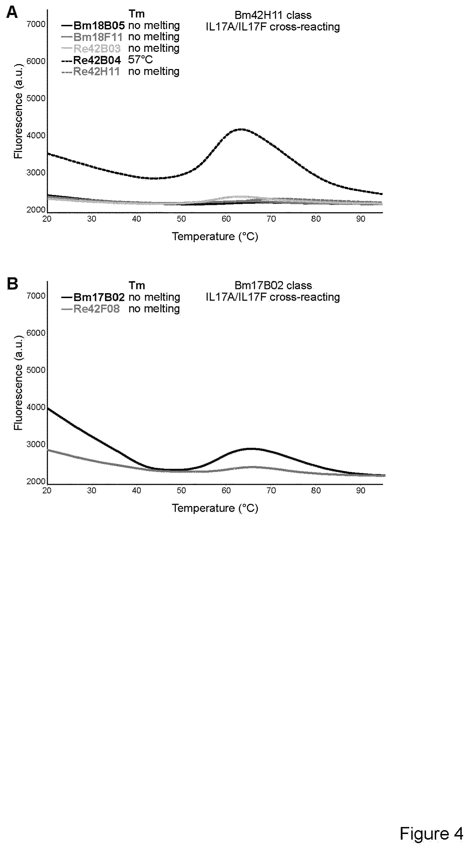

**Figure 4****: Thermal stability of disclosed VHH antibodies.**
   VHH antibodies were subjected to Differential scanning fluorimetry (DSF), which exploits that thermal unfolding exposes aromatic/hydrophobic residues, which then bind and). The plate was sealed with transparent MicroSeal^{®} 'B' Seal (Bio-Rad), briefly centrifuged to remove any air bubbles, and placed onto a CFX96 Real-Time System (C1000 Thermal Cycler, BioRad). The samples were incubated for 5 min at 20°C. The temperature was then increased by 1°C every 45 seconds until 95°C was reached. Fluorescence was measured at the end of each step with 532 nm excitation and a 555 nm long pass filter. Melting temperatures are defined as the inflection point of the first melting peak. The very low melting amplitudes of Re42H11, Re42B03, Bm18F11, Bm18B05, and Re42F08 indicate resistance to melting and, thus, full thermal stability. For controls under reducing (disulfide bond-breaking) conditions, see Figure 5.
**Figure 5****: Thermal stabilities under non-reducing and disulfide bond-reducing conditions.**
   Thermal stability assays were performed as in Figure 4. Here, however, controls in the presence of 10 mM dithiothreitol (DTT) are shown. DTT reduces the structural, stabilizing disulfide bond. Note the far higher unfolding amplitude in the presence of DTT. These controls indicate which fluorescence signal to expect if a VHH antibody would melt completely.

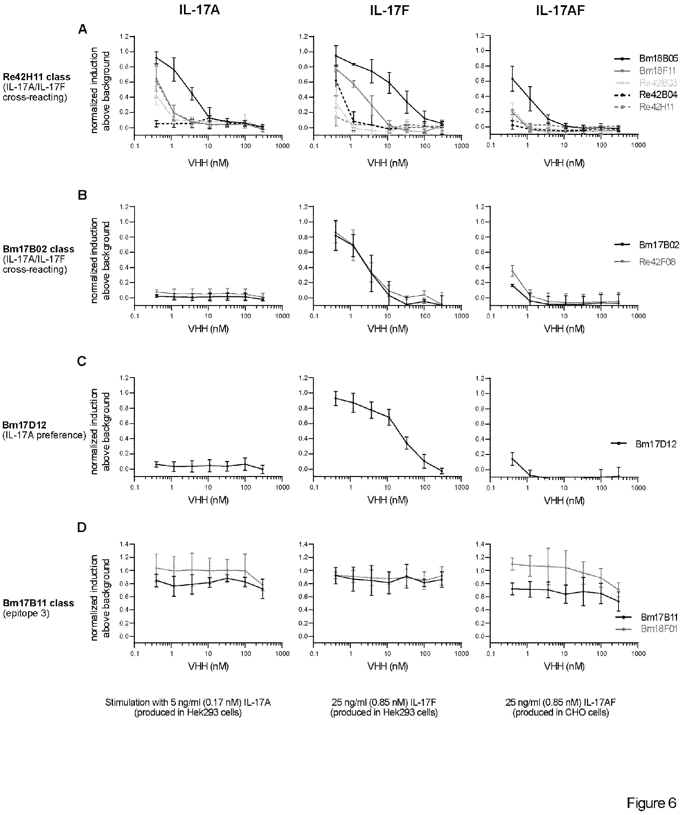

**Figure 6****: Neutralization of IL-17A and IL-17F by the Re42H11 class of VHH antibodies.**
   Plots show the expression of a phosphatase reporter induced by 0.17 nM IL-17A homodimers, 0.85 nM IL-17F homodimers, or 0.85 nM IL-17AF heterodimers preincubated with indicated concentrations of anti-IL-17 VHH antibodies. A number of 1.0 indicates (full) induction in the absence of a VHH, and a number of 0 indicates no induction above the background of the minus VHH controls. Graphs show neutralization by Re42H11 and Bm17B02 class members, each measured in triplicates. Note the excellent neutralization at low nanomolar or even sub-nanomolar concentrations. Bm17D12 is a control VHH that binds IL-17A tightly but IL-17F only weakly. Bm17B11 and Bm18F01 bind only IL-17A, and this at the non-neutralizing epitope 3.

Nanobody neutralization assays were carried out using Hek-Blue^{™} IL-17 cells (Invivogen hkb-il17) according to the manufacturer's instructions. These cells are Hek293 cells stably expressing the human IL-17RA and IL-17RC receptors as well as the adapter Act1. Hek-Blue^{™} IL-17 cells further encode a secreted embryonic alkaline phosphatase (SEAP) reporter controlled by a promoter with NF-kB and AP-1 binding sites. Upon binding of the IL-17 ligands to its receptors on the cell surface, NF-kB and AP-1 get activated, SEAP is produced and secreted into the cell supernatant. The amount of SEAP is monitored colorimetrically, using QUANTI-Blue^{™} as a substrate. Note the efficient block of reporter production by the added VHH antibodies.

### Examples

The generation of anti-IL-17 VHH antibodies showing a high-affinity cross-reaction between several different IL-17 isoforms was a highly challenging undertaking - given that the sequence identity between the IL-17 paralogues is limited to only ~50% identity between human IL-17A and IL-17F and that most conserved residues are actually buried in the hydrophobic core of the disulfide-bridged IL-17 dimer. The desired VHH antibody would thus likely have to tolerate sequences exchanges in its epitope without loss of binding strength. The IL-17 receptors can tolerate such sequence variability more easily because they have an interaction interface that is much larger than the epitope of a VHH antibody can possibly be.

The inventors, therefore, considered these constraints in their VHH generation strategy. They recombinantly produced the human IL-17A homodimer, the IL-17F homodimer, and the IL-17A-F heterodimer and injected the three multiple times as immunogens into an alpaca. This strategy was chosen to foster an immune response comprising also antibodies that cross-react between the IL-17 paralogues.

After the final immunization, a blood sample was taken, lymphocytes were isolated, and mRNA was purified and reverse transcribed. VHH-coding regions were amplified by nested PCR, reverse transcribed, and cloned as cDNAs into an M13 phagemid, yielding a library with >100 million independent clones.

In the next steps, several rounds of phage display were performed. Selected VHH antibodies were classified according to sequence similarity, cloned into *E. coli* expression vectors, produced by periplasmic expression, purified, and finally characterized for binding of IL-17 using biolayer interferometry (BLI; Abdiche *et al.,* 2008).

In one arm of the selection approach, the IL-17A homodimer and the IL-17A/F heterodimer were used successively as baits. This yielded a considerable sequence diversity in the selected VHH antibodies - approximately 8 major classes and numerous minor ones as judged by sequence similarities. Most tested ones bound IL-17A with sub-nanomolar affinities. 'Binning' identified three complementary epitopes (epitope 1-3), i.e., epitope 1-binding VHH antibodies could bind simultaneously with either epitope 2-binders or epitope 3-binders. Likewise, epitope 2- and 3-binders could also bind simultaneously.

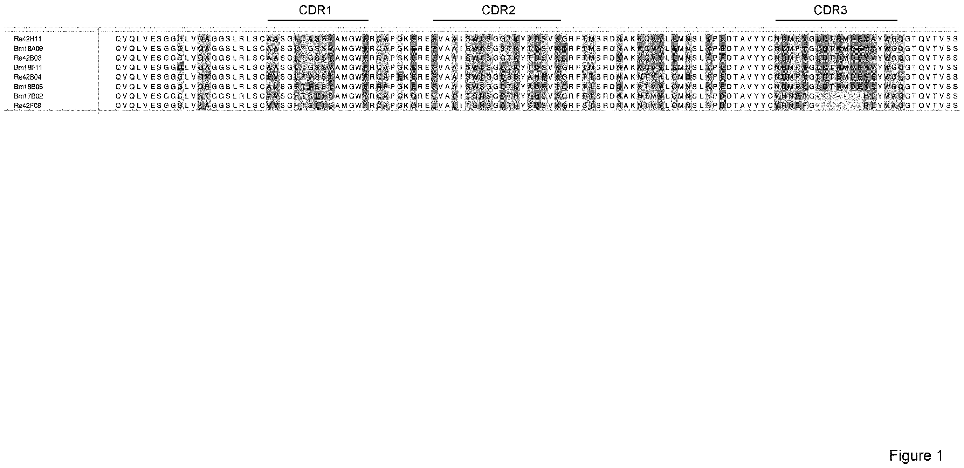

In the second selection arm, the IL-17A homodimer, the IL-17F homodimer, and the IL-17A/F heterodimer were used successively as baits. This direct selection for crossreactivity reduced the complexity of sequences considerably. All epitope 2- and epitope 3-binders were lost. Two VHH classes became dominant and accounted now for 98% of all sequences. These two classes are disclosed in the following. We refer to them as the Re42H11 and the Bm17B02 classes - named after VHH antibodies that represent the respective class consensus (Figure 1).

Five members of the Re42H11 class (Re42H11 itself, Re42B03, Re42B04, Re42B05, and Bm18F11), as well as two Bm17B02 class members (Bm17B02 and Re42F08), were characterized in depth. For affinity measurements, they were produced with a C-terminal Avi-Biotin-tag and immobilized on High precision streptavidin sensors of an Octet RED96e BLI instrument.

These measurements revealed perfect cross-reactivities with IL-17A, IL-17F, and IL17-A/F heterodimer (Figures 2 and 3; Table 1), high on-rates (mostly around 10⁵ - 10⁶ M^{-1.}s⁻¹), non-detectable or nearly non-detectable dissociation rates, and extremely high affinities of 10 pM or better (only the Bm17B02 IL-17F affinity pair showed in these measurements a higher KD of 70 pM). Note that the BLI setup does not allow to discern affinities below 10 pM.

For the intended therapeutic application, the IL-17 VHH antibodies should not only bind their targets with high affinity, but also, they should be developable as biological drugs. This includes that they are stable enough to survive a lengthy, large-scale production process as well as transportation and storage (ideally for years in liquid formulation) without aggregation or loss of activity.

A good predictor for stability is thermostability, which can be measured, e.g., by thermal shift assays or specifically by differential scanning fluorimetry (Goldberg *et al.,* 2011). The method exploits that melting (thermal unfolding) of a protein exposes aromatic/hydrophobic residues (from its hydrophobic core), which then bind and enhance the fluorescence of the added SYPRO Orange dye. Figure 4 shows such analyses for the disclosed VHH antibodies, with slow heating from 20°C to 95°C. The majority of VHHs (Re42H11 itself, Re42B03, Re42B04, Re42B05, Bm18F11, as well as Re42F08) showed only a negligible unfolding signal and thus no melting (the small fluorescence peak did not or did hardly exceed the measured fluorescence at 20°C). As a control, the inventors repeated the experiment for some candidates in the presence of DTT (dithiothreitol) to reduce the structural disulfide bond and now, they observed large unfolding peaks that indicate a global melting of the respective VHH antibodies (Figure 5). One Re42H11 class member (Re42B04) showed a small melting peak that exceeded the 20°C-signal and had an inflection point at 57°C (Figure 4); however, not even this VHH aggregated upon heating to 95°C. Thus, the heredisclosed anti-IL-17 VHH antibodies fulfill three key criteria for the intended application: extremely high affinity for their target, perfect cross-reaction between IL17A, IL17A/F, and IL17F, as well as extraordinarily high thermal stability.

To assess if the disclosed anti-IL17 VHH antibodies block the binding of IL-17 to its receptors, the inventor carried out cell-based neutralization assays using Hek-Blue^{™} IL-17 cells. These cells express human IL-17RA and IL-17RC receptors as well as the adapter Act1. They secrete a phosphate reporter in response to the receptor binding of IL-17 (Figure 6). By quantitating the amount of secreted phosphatase, the activation of the IL17 receptors can be measured. The data show that a pre-incubation of IL-17A or IL-17F with any of Re42H11 or Bm17B02 class members impeded the receptor activation. Remarkably, IL-17A neutralization at sub-nanomolar concentrations was observed, e.g., for Re42B04, Re42B03, Bm17D12, and Re42F08. Likewise, the IL-17A/F heterodimer was neutralized potently whenever the VHHs Bm18F11, Re42B03, Re42B04, Re42H11, Bm17B02, Re42F08, or Bm17D12 exceeded the interleukin concentration. Re42H11, Re42B03, and Re42B04 were particularly efficient in blocking the IL-17F homodimer, showing a perfectly stoichiometric neutralization.

### IL-17 sequences

>IL-17A homodimer (SEQ. ID NO: 1) 
GSEDSNFPRTVMVNLNIHNRNTNTNPKRSSDYYDRSTSPWNLHRNEDPERYPSVI
WEAKCRHLGCINADGNVDYHMNSVPIQQEILVLRREPPHSPNSFRLEKILVSVGCTC
VTPIVHHVA
>IL-17F homodimer (SEQ. ID NO: 2) 
GSEGETFFQKPESCPPVPGGSMKLDIGIINENQRVSMSRNIESRSTSPWDYTVTWD
PNRYPSEVVQAQCRNLGCINAQGKEDISMNSVPIQQETLVVRRKHQGCSVSFQLEK
VLVTVGCTCVTPVIHHVQ
 IL-17A/F heterodimer - two polypeptide units
>IL-17A unit (SEQ. ID NO: 3) 
GSEDSNFPRTVMVNLNIHNRNTNTNPKRSSDYYDRSTSPWNLHRNEDPERYPSVI
WEAKCRHLGCINADGNVDYHMNSVPIQQEILVLRREPPHCPNSFRLEKILVSVGCTC
VTPIVHHVA
>IL-17F unit (SEQ. ID NO: 4)

GSEGETFFQKPESCPPVPGGSMKLDIGIINENQRVSMSRNIESRSTSPWDYTVTWD
PNRYPSEVVQAQCRNLGCINAQGKEDISMNSVPIQQETLVVRRKHQGSSVSFQLEK
VLVTVGCTCVTPVIHHVQ

### VHH antibody sequences

>Re42H11 SEQ. ID NO: 5) 
QVQLVESGGGLVQAGGSLRLSCAASGLTASSYAMGWFRQAPGKEREFVAAISWIS
GGTKYADSVKGRFTMSRDNAKKQVYLEMNSLKPEDTAVYYCNDMPYGLDTRMDE
YAYWGQGTQVTVSS
>Bm18A09 SEQ. ID NO: 9) 
QVQLVESGGGLVQAGGSLRLSCAASGLTGSSYAMGWFRQAPGKEREFVAAISWIS
GSTKYTDSVKDRFTMSRDNAKKQVYLEMNSLKPEDTAVYYCNDMPYGLDTRMDEY
VYWGQGTQVTVSS
>Re42B03 SEQ. ID NO: 13 
QVQLVESGGGLVQAGGSLRLSCAASGLTGSSYAMGWFRQAPGKEREFVAAISWIS
GGTKYTDSVKDRFTMSRDYAKKQVYLEMNSLKPEDTAVYYCNDMPYGLDTRMDE
YVYWGQGTQVTVSS
>Re42B04 (SEQ. ID NO: 17) 
QVQLVESGGGLVQVGGSLRLSCEVSGLPVSSYAMGWFRQAPEKEREFVAAISWIG
GDSRYAHFVKGRFTISRDNAKNTVHLQMDSLKPEDTAVYYCNDMPYGLDTRMDEY
EYWGLGTQVTVSS
>Bm18F11 (SEQ. ID NO: 21) 
QVQLVESGGDLVQAGGSLRLSCAASGLTGSSYAMGWFRQAPGKEREFVAAISWIS
GDTKYTDSVKGRFTMSRDNAKKQVYLEMNSLKPEDTAVYYCNDMPYGLDTRMDE
YVYWGQGTQVTVSS
>Bm18B05 (SEQ. ID NO: 25) 
QVQLVESGGGLVQPGGSLRLSCAVSGRTFSSYAMGWFRRPPGKEREFVAAISWS
GGDTKYADFVTDRFTISRDDAKSTVYLQMNSLKPEDTAVYYCNDMPYGLDTRMDE
YEYWGQGTQVTVSS
>Bm17B02 SEQ. ID NO: 29) 
QVQLVESGGGLVNTGGSLRLSCVVSGHTSEISAMGWYRQAPGKQRELVALITSRS
GDTHYSDSVKGRFSISRDNAKNTMYLQMNSLNPDDTAVYYCVHNEPGHLYMAQGT
QVTVSS
>Re42F08 (SEQ. ID NO: 33)

QVQLVESGGGLVKAGGSLRLSCVVSGHTSEISAMGWYRQAPGKQRELVALITSRS
GDTHYSDSVKGRFSISRDNAKNTMYLQMNSLNPDDTAVYYCVHNEPGHLYMAQGT
QVTVSS

### References

Abdiche Y, Malashock D, Pinkerton A, Pons J (2008) Determining kinetics and affinities of protein interactions using a parallel real-time label-free biosensor, the Octet. Anal Biochem, 377: 209-217
Beckett D, Kovaleva E, Schatz PJ (1999) A minimal peptide substrate in biotin holoenzyme synthetase-catalyzed biotinylation. Protein Sci, 8: 921-929
Glatt S, Baeten D, Baker T, Griffiths M, Ionescu L, Lawson ADG, Maroof A, Oliver R, Popa S, Strimenopoulou F, Vajjah P, Watling MIL, Yeremenko N, Miossec P, Shaw S (2018) Dual IL-17A and IL-17F neutralisation by bimekizumab in psoriatic arthritis: evidence from preclinical experiments and a randomised placebo-controlled clinical trial that IL-17F contributes to human chronic tissue inflammation. Ann Rheum Dis, 77: 523-532
Goldberg DS, Bishop SM, Shah AU, Sathish HA (2011) Formulation development of therapeutic monoclonal antibodies using high-throughput fluorescence and static light scattering techniques: role of conformational and colloidal stability. J Pharm Sci, 100: 1306-1315
Güttler T, Aksu M, Dickmanns A, Stegmann KM, Gregor K, Rees R, Taxer W, Rymarenko O, Schünemann J, Dienemann C, Gunkel P, Mussil B, Krull J, Teichmann U, Groß U, Cordes VC, Dobbelstein M, Görlich D (2021) Neutralization of SARS-CoV-2 by highly potent, hyperthermostable, and mutation-tolerant nanobodies. EMBO J, e107985
Langley RG, Elewski BE, Lebwohl M, Reich K, Griffiths CE, Papp K, Puig L, Nakagawa H, Spelman L, Sigurgeirsson B, Rivas E, Tsai TF, Wasel N, Tyring S, Salko T, Hampele I, Notter M, Karpov A, Helou S, Papavassilis C, ERASURE SG, FIXTURE SG (2014) Secukinumab in plaque psoriasis--results of two phase 3 trials. N Engl J Med, 371: 326-338
Mease PJ, McInnes IB, Kirkham B, Kavanaugh A, Rahman P, van der Heijde D, Landew6 R, Nash P, Pricop L, Yuan J, Richards HB, Mpofu S, FUTURE SG (2015) Secukinumab Inhibition of Interleukin-17A in Patients with Psoriatic Arthritis. N Engl J Med, 373: 1329-1339
Mills KHG (2022) IL-17 and IL-17-producing cells in protection versus pathology. Nat Rev Immunol, 1-17
Nies JF, Panzer U (2020) IL-17C/IL-17RE: Emergence of a Unique Axis in TH17 Biology. Front Immunol, 11: 341
Papp KA, Weinberg MA, Morris A, Reich K (2021) IL17A/F nanobody sonelokimab in patients with plaque psoriasis: a multicentre, randomised, placebo-controlled, phase 2b study. Lancet, 397: 1564-1575
Reich K, Warren RB, Lebwohl M, Gooderham M, Strober B, Langley RG, Paul C, De Cuyper D, Vanvoorden V, Madden C, Cioffi C, Peterson L, Blauvelt A (2021) Bimekizumab versus Secukinumab in Plaque Psoriasis. N Engl J Med, 385: 142-152
Skroza N, Proietti I, Bernardini N, Aquila E, Balduzzi V, La Viola G, Mambrin A, Muscianese M, Tolino E, Zuber S, Potenza C, Ferrazza P (2017) II-17 and Its Role in Psoriasis, Hidradenitis Suppurativa And Acne. Internal Medicine and Care, 1**:**
Warren RB, Blauvelt A, Bagel J, Papp KA, Yamauchi P, Armstrong A, Langley RG, Vanvoorden V, De Cuyper D, Cioffi C, Peterson L, Cross N, Reich K (2021) Bimekizumab versus Adalimumab in Plaque Psoriasis. N Engl J Med, 385: 130-141
Wilson SC, Caveney NA, Yen M, Pollmann C, Xiang X, Jude KM, Hafer M, Tsutsumi N, Piehler J, Garcia KC (2022) Organizing structural principles of the IL-17 ligandreceptor axis. Nature, 609: 622-629

## Claims

1. A VHH antibody recognizing a human IL-17 polypeptide,
which cross-reacts with a plurality of different human IL-17 polypeptides comprising (i) a human IL-17A homodimer, (ii) a human IL-17F homodimer and (iii) a human IL17A/F heterodimer wherein the binding affinity expressed as dissociation constant KD to each of (i), (ii), and (iii) is about 1 nM or less, about 100 pM or less, about 50 pM or less, about 20 pM or less or about 10 pM or less.

2. The VHH antibody according to claim 1,
which competes with the VHH antibody Re42H11 having a VHH sequence as shown in SEQ. ID NO: 5 for the binding to a human IL-17 polypeptide, or with the VHH antibody Bm17B02 having a VHH sequence as shown in SEQ. ID NO: 29 for the binding to a human IL-17 polypeptide.

3. A VHH antibody recognizing a human IL-17 polypeptide, comprising
(a) a CDR3 sequence as shown in SEQ. ID NO: 6, 10, 14, 18, 22, 26, 30 and 34;
(b) a CDR3 sequence, which has an identity of at least 80%, at least 90% or at least 95% to a CDR3 sequence of (a), or
(c) a VHH antibody, which competes with the VHH antibody Re42H11 having a VHH sequence as shown in SEQ. ID NO: 5 for the binding to a human IL-17 polypeptide, or which competes with the VHH antibody Bm17B02 having a VHH sequence as shown in SEQ. ID NO: 29 for the binding to a human IL-17 polypeptide.

4. The VHH antibody according to claim 3 comprising
(a) a combination of CDR1, CDR2 and CDR3 sequences as shown in SEQ. ID NO: 6-8, 10-12, 14-16, 18-20, 22-24, 26-28, 30-32, and 34-36,
(b) a combination of CDR1, CDR2 and CDR3 sequences which has an identity of at least 80%, at least 90% or at least 95% to a combination of CDR1, CDR2 and CDR3 sequences of (a), or
(c) a VHH antibody, which competes with the VHH antibody Re42H11 having a VHH sequence as shown in SEQ. ID NO: 5 for the binding to a human IL-17 polypeptide, or which competes with the VHH antibody Bm17B02 having a VHH sequence as shown in SEQ. ID NO: 29 for the binding to a human IL-17 polypeptide.

5. The VHH antibody according to any one of the previous claims, comprising
(a) a VHH sequence as shown in SEQ. ID NO: 5, 9, 13, 17, 21, 25, 29, or 33;
(b) a sequence which has an identity of at least 80%, at least 90%, at least 95% or at least 99% to a VHH sequence of (a), or
(c) a VHH antibody, which competes with the VHH antibody Re42H11 having a VHH sequence as shown in SEQ. ID NO: 5 for the binding to a human IL-17 polypeptide, or which competes with the VHH antibody Bm17B02 having a VHH sequence as shown in SEQ. ID NO: 29 for the binding to a human IL-17 polypeptide.

6. The VHH antibody of any one of claims 3-5, which cross-reacts with (i) an IL-17A homodimer, (ii) an IL-17F homodimer and (iii) an IL17A/F heterodimer, wherein the binding affinity expressed as dissociation constant KD to each of (i), (ii), and (iii) is about 1 nM or less, about 100 pM or less, about 50 pM or less, about 20 pM or less or about 10 pM or less.

7. The VHH antibody of any one of the preceding claims, which neutralizes the binding of (i) a human IL-17A homodimer, (ii) a human IL-17F homodimer and (iii) a human IL17A/F heterodimer to a human IL-17 receptor, particularly at a concentration of about 10 nM or less, of about 1 nM or less, or of about 0.5 nM or less.

8. The VHH antibody of any one of the preceding claims, which is stable, particularly thermostable or hyperthermostable, which has a melting temperature of at least about 65°C, of at least about 80°C, of at least 90°C or of at least about 95°C when measured under non-reducing conditions.

9. The VHH antibody of any one of claims 1-8, which is selected from:
- antibody Re42H11 comprising a VHH sequence as shown in SEQ. ID NO: 5 or a VHH antibody, which is a variant thereof;
- antibody Bm18A09 comprising a VHH sequence as shown in SEQ. ID NO: 9 or a VHH antibody, which is a variant thereof;
- antibody Re42B03 comprising a VHH sequence as shown in SEQ. ID NO: 13 or a VHH antibody, which is a variant thereof;
- antibody Re42B04 comprising a VHH sequence as shown in SEQ. ID NO: 17 or a VHH antibody, which is a variant thereof;
- antibody Bm18F11 comprising a VHH sequence as shown in SEQ. ID NO: 21 or a VHH antibody, which is a variant thereof;
- antibody Bm18B05 comprising a VHH sequence as shown in SEQ. ID NO: 25 or a VHH antibody, which is a variant thereof;
- antibody Bm17B02 comprising a VHH sequence as shown in SEQ. ID NO: 29 or a VHH antibody, which is a variant thereof; and
- antibody Re42F08 comprising a VHH sequence as shown in SEQ. ID NO: 33 or a VHH antibody, which is a variant thereof.

10. The VHH antibody of any one of claims 1-9 for use in the prevention or treatment of a disorder caused by and/or associated with an overactivity of IL-17, particularly with an overactivity of IL-17A and/or IL-17F.

11. The VHH antibody of any one of claims 1-9 for use in the prevention or treatment of an inflammatory and/or immune-related disorder, particularly for use in the prevention or treatment of an inflammatory and/or immune-related skin disorder.

12. The VHH antibody of any one of claims 1-9 for use in the prevention or treatment of asthma, psoriasis, e.g., plaque psoriasis, arthritis, e.g., rheumatoid arthritis or psoriatic arthritis, Hidradenitis suppurativa, inflammatory bowel disease (Crohn's disease, ulcerative colitis), multiple sclerosis, skin cancer, ankylosing spondylitis, uveitis, atopic dermatitis, Graft versus Host Disease, Alzheimer's disease, fatty liver disease, COVID-19, sepsis, ischemic stroke, Parkinson's disease, influenza virus infection.

13. The VHH antibody of any one of claims 1-9 in a human subject.

14. The VHH antibody of any one of claims 1-9, which is administered locally, e.g., topically.

15. A pharmaceutical composition comprising as an active agent a VHH antibody of any one of claims 1-9, and a pharmaceutically acceptable carrier.
